# EUROPÄISCHE PATENTSCHRIFT

(11) **EP 3 166 588 B1**
(45) Veröffentlichungstag und Bekanntmachung des Hinweises auf die Patenterteilung: **14.08.2019**
(21) Anmeldenummer: 15731588.8
(22) Anmeldetag: 25.06.2015
(51) Int. Cl.: A61K 8/64, A61Q 15/00

(54) **VERWENDUNG EINES SPEZIELLEN PROTEINS ZUR VERMINDERUNG DER TRANSPIRATION DES KÖRPERS**
USE OF A SPECIFIC PROTEIN TO REDUCE TRANSPIRATION OF THE BODY
UTILISATION D'UNE PROTEINE SPECIALE POUR REDUIRE LA TRANSPIRATION DU CORPS

(30) Priorität: 08.07.2014 DE 102014213223
(43) Veröffentlichungstag der Anmeldung: 17.05.2017
(73) Patentinhaber: Henkel AG & Co. KGaA, 40589 Düsseldorf (DE)
(72) Erfinder: BANOWSKI, Bernhard, 40597 Düsseldorf (DE); EVERS, Stefan, 42781 Haan (DE)
(86) Internationale Anmeldenummer: PCT/EP2015/064410
(87) Internationale Veröffentlichungsnummer: WO 2016/005198

(56) Entgegenhaltungen:
- EP-A1- 2 143 418
- EP-A2- 1 813 310
- WO-A1-94/24993
- WO-A1-2008/025698
- WO-A1-2010/003861
- US-A1- 2012 006 348
- DATABASE GNPD [Online] MINTEL; August 2006 (2006-08), "Deodorant Cream", XP002743988, Database accession no. 568915
- DATABASE GNPD [Online] MINTEL; August 2013 (2013-08), "24h Anti-Perspirant Deodorant Spray", XP002743989, Database accession no. 2151537
- DATABASE GNPD [Online] MINTEL; April 2014 (2014-04), "Deodorant", XP002743990, Database accession no. 2371606

## Beschreibung

Die vorliegende Erfindung betrifft die Verwendung von Milchproteinen und/oder Joghurtproteinen und/oder Seidenproteinen zur zumindest teilweisen Beeinflussung der Schweißdrüse(n).

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Im Stand der Technik sind zahlreiche spezielle deodorierende oder schweißhemmende Körperpflegemittel bekannt, welche für die Anwendung in Körperregionen mit einer hohen Dichte von Schweißdrüsen, insbesondere im Achselbereich, entwickelt wurden. Diese sind in den unterschiedlichsten Darreichungsformen konfektioniert, beispielsweise als Puder, in Stiftform, als Aerosolspray, Pumpspray, flüssige und gelförmige Roll-on-Applikation, Creme, Gel und als getränkte flexible Substrate (Deotücher).

Kosmetische Antitranspirantien des Standes der Technik enthalten neben mindestens einem Öl oder einem Wachs und einer Riechstoffkomponente bzw. einem Parfüm mindestens eine schweißhemmende Verbindung, insbesondere in Form von Halogeniden und/oder Hydroxyhalogeniden von Aluminium und/oder Zirconium. Diese schweißhemmenden Verbindungen verringern zum einen die Schweißsekretion des Körpers durch eine temporäre Verengung und/oder Verstopfung der Ausführungsgänge der Schweißdrüsen, so dass die Schweißmenge um etwa 20 bis 60 Prozent reduziert werden kann. Zum anderen weisen sie aufgrund ihrer antimikrobiellen Wirkung einen zusätzlichen desodorierenden Effekt auf.

Halogenide und/oder Hydroxyhalogenide von Aluminium und/oder Zirconium können in Verbindung mit dem sauren pH-Wert dieser Antitranspirantien bei einigen Anwendern zu unangenehmen Hautreaktionen führen. Darüber hinaus kann die Verwendung der vorgenannten schweißhemmenden Verbindungen zu einer Fleckenbildung auf der Kleidung führen.

Es besteht daher ein Bedarf schweißhemmende Halogenide und/oder Hydroxyhalogenide von Aluminium und/oder Zirconium gegen andere schweißhemmende kosmetische Wirkstoffe auszutauschen. Diese schweißhemmenden Wirkstoffe sollen eine gute schweißhemmende Wirkung, eine gute Hautverträglichkeit sowie eine einfache Formulierbarkeit aufweisen. Darüber hinaus sollen diese schweißhemmenden Wirkstoffe keinen negativen Einfluss auf die Lagerstabilität der schweißhemmenden kosmetischen Mittel aufweisen.

In Dokument WO 2010/003861 wird die Verwendung von Proteinen und/oder Proteinhydrolysaten als schweißhemmende Wirkstoffe vorgeschlagen.

In dem Dokument WO 2008/025698 werden Antitranspirant- und Deodorant-Zusammensetzungen offenbart, die als schweißhemmende Wirkstoffe Aluminium- und/oder Zirkoniumsalze sowie zusätzlich Yoghurt-Proteine und/oder deren Hydrolysate zur Vermeidung von Hautreizungen enthalten.

Der vorliegenden Erfindung lag die Aufgabe zugrunde alternative Wirkstoffe mit schweißhemmender Wirkung bereitzustellen.

Es wurde nun überraschend gefunden, dass die Verwendung mindestens eines Milchproteins und/oder Joghurtproteins und/oder Seidenproteins in kosmetischen Mitteln ohne schweißhemmende Halogenide und/oder Hydroxyhalogenide von Aluminium und/oder Zirconium in einer schweißhemmenden Wirkung resultiert, welche mit der schweißhemmenden Wirkung von Formulierungen mit Aluminiumsalzen und/oder Aluminium-Zirkonium-Komplexen nahezu vergleichbar ist.

Durch den Einsatz des mindestens eines Milchproteins und/oder Joghurtproteins und/oder Seidenproteins in kosmetischen Mitteln erfolgt - ohne sich auf diese Theorie beschränken zu wollen - eine gezielte Beeinflussung der Schweißdrüse(n). Diese gezielte Beeinflussung der Schweißdrüse(n) kann beispielsweise in einer Gelbildung des mindestens einen Milchproteins und/oder Joghurtproteins und/oder Seidenproteins bei pH-Werten, welche ausschließlich innerhalb der Ausführungsgänge der Schweißdrüsen vorliegen, bestehen. Auf diese Art und Weise kann eine effektive Verstopfung der Ausführungsgänge der Schweißdrüsen gewährleistet werden, ohne dass die schweißhemmende Wirkung des kosmetischen Mittels durch vorzeitige unerwünschte Gelbildung aufgrund des Zusatzes des mindestens einen speziellen Milchproteins und/oder Joghurtproteins und/oder Seidenproteins vermindert wird. Weiterhin kann die gezielte die Beeinflussung der Schweißdrüse(n) jedoch auch in einer Störung des Ladungsgleichgewichts innerhalb der Schweißdrüse(n) bestehen, welche zu einer Beeinflussung der Schweißproduktion, insbesondere zu einer Verminderung der Schweißproduktion, führt. Somit wird selbst bei Abwesenheit schweißhemmender Halogenide und/oder Hydroxyhalogenide von Aluminium und/oder Zirconium eine effektive Verminderung des Achselschweißes gewährleistet.

Unter dem Begriff "schweißhemmend" wird erfindungsgemäß die Verminderung bzw. Reduzierung der Transpiration der Schweißdrüsen des Körpers verstanden.

Darüber hinaus werden unter dem Begriff "Halogenide und/oder Hydroxyhalogenide von Aluminium und/oder Zirconium" im Rahmen der vorliegenden Erfindung insbesondere Chloride, Bromide und lodide des Aluminiums und des Zirconiums sowie Verbindungen der Formeln Al(OH)yX und Zr(OH)_{z}X verstanden, wobei X in den vorgenannten Formeln für ein Halogenidion steht.

Weiterhin wird unter dem Begriff "kosmetisches Öl" im Sinne der vorliegenden Erfindung ein für die kosmetische Verwendung geeignetes Öl verstanden, welches mit Wasser nicht in allen Mengen mischbar ist. Bei dem kosmetischen Öl handelt es sich weder um Riechstoffe, noch um ätherische Öle.

Zudem werden unter dem Begriff "Riechstoffe" im Sinne der vorliegenden Erfindung Substanzen mit einer Molmasse von 74 bis 300 g/mol verstanden, welche mindestens eine osmophore Gruppe im Molekül enthalten und einen Geruch und/oder Geschmack aufweisen, d. h. sie sind in der Lage, die Rezeptoren der Haarzellen des olfaktorischen Systems zu erregen. Osmophore Gruppen sind kovalent an das Molekülgerüst gebundene Gruppen in Form von Hydroxygruppen, Formylgruppen, Oxogruppen, Alkoxycarbonylgruppen, Nitrilgruppen, Nitrogruppen, Azidgruppen etc. In diesem Zusammenhang fallen unter den Begriff "Riechstoffe" im Sinne der vorliegenden Erfindung auch bei 20 °C und 1.013 hPa flüssige Parfümöle, Parfüme, oder Parfümölbestandteile,

Darüber hinaus werden unter dem Begriff "Wachse" im Rahmen der vorliegenden Erfindung Substanzen verstanden, welche bei 20 °C knetbar oder fest bis brüchig hart sind, eine grobe bis feinkristalline Struktur aufweisen und farblich durchscheinend bis opak, aber nicht glasartig sind. Weiterhin schmelzen diese Substanzen über 25 °C ohne Zersetzung, sind wenig oberhalb des Schmelzpunktes leicht flüssig (wenig viskos), weisen eine stark temperaturabhängige Konsistenz und Löslichkeit auf und sind unter leichtem Druck polierbar.

Der Begriff "Protein" bezeichnet erfindungsgemäß chemische Verbindungen, welche durch Peptid-Bindungen Säureamid-artig verknüpfte Kondensationsprodukte von Aminosäuren bilden. Die Anzahl der Aminosäuren in den Proteinen beträgt bevorzugt mindestens 2 und maximal 1.000 Aminosäuren. Unter dem Begriff "Protein" sind erfindungsgemäß auch Hydrolysate eines Proteins zu verstehen, welche Proteinfraktionen mit verschiedenen Aminosäuresequenzen und Molekulargewichten enthalten. Darüber hinaus sind unter diesem Begriff im Rahmen der vorliegenden Erfindung auch Mischungen von in tierischen Sekreten, Sekreten von Insekten oder humanen Sekreten vorkommenden Proteinen zu verstehen.

Darüber hinaus wird unter dem Begriff "Veränderung der Lichtabsorption des mindestens einen Proteins" sowohl die positive und negative Veränderung der Lichtdurchlässigkeit der Probenmischung, insbesondere der Proteinlösung, als auch die Absorption von Licht durch das mindestens eine Protein bzw. die Probenmischung verstanden.

Weiterhin wird unter dem Begriff "pH-Wert Änderung" die kontinuierliche Veränderung des pH-Wertes verstanden. Die kontinuierliche Veränderung des pH-Wertes kann beispielsweise durch Titration, bzw. gleichmäßige Zugabe, einer Base oder Säure erreicht werden.

Der Begriff "Probenmischung" bezeichnet erfindungsgemäß eine Mischung aus dem mindestens einen speziellen Milchprotein und/oder Joghurtprotein und/oder Seidenprotein mit einem Lösungsmittel, insbesondere Wasser, Puffer oder salzhaltigen wässrigen Lösungen.

Zudem sind unter dem Begriff der "Fettsäuren", wie er im Rahmen der vorliegenden Erfindung verwendet wird, aliphatische Carbonsäuren zu verstehen, welche unverzweigte oder verzweigte Kohlenstoffreste mit 4 bis 40 Kohlenstoffatomen aufweisen. Die im Rahmen der vorliegenden Erfindung eingesetzten Fettsäuren können sowohl natürlich vorkommende als auch synthetisch hergestellte Fettsäuren sein. Weiterhin können die Fettsäuren einfach oder mehrfach ungesättigt sein.

Schließlich sind unter dem Begriff der "Fettalkohole" im Rahmen der vorliegenden Erfindung aliphatische, einwertige, primäre Alkohole zu verstehen, welche unverzweigte oder verzweigte Kohlenwasserstoffreste mit 4 bis 40 Kohlenstoffatomen aufweisen. Die im Rahmen der Erfindung eingesetzten Fettalkohole können auch ein- oder mehrfach ungesättigt sein.

Die Angabe Gew.-% bezieht sich vorliegend, sofern nichts anderes angegeben ist, auf das Gesamtgewicht der schweißhemmenden kosmetischen Mittel.

Milchproteine und/oder Joghurtproteine und/oder Seidenproteine können vorzugsweise in schweißhemmenden kosmetischen Mitteln verwendet werden, die mindestens einen Stoff a) enthalten, welcher aus der Gruppe von kosmetischen Ölen, welche bei 20 °C und 1.013 hPa flüssig sind, Riechstoffen und Wachsen, ausgewählt ist.

Im Rahmen der vorliegenden Erfindung ist das bei 20°C und 1.013 hPa flüssige kosmetische Öl ausgewählt aus der Gruppe von (i) flüchtigen cyclischen Siliconölen, insbesondere cylischen und linearen Siliconölen; (ii) flüchtigen Nichtsiliconölen, insbesondere flüssigen Paraffinölen und Isoparaffinölen; (iii) nichtflüchtigen Siliconölen; (iv) nichtflüchtigen Nichtsiliconölen; sowie (v) deren Mischungen.

Der Begriff "flüchtiges Öl" bezeichnet erfindungsgemäß Öle, welche bei 20 °C und einem Umgebungsdruck von 1.013 hPa einen Dampfdruck von 2,66 Pa bis 40.000 Pa (0,02 bis 300 mm Hg), vorzugsweise von 10 bis 12.000 Pa (0,1 bis 90 mm Hg), weiter bevorzugt von 13 bis 3.000 Pa (0,1 bis 23 mm Hg), insbesondere von 15 bis 500 Pa (0,1 bis 4 mm Hg), aufweisen.

Darüber hinaus werden unter dem Begriff "nichtflüchtige Öle" im Sinne der vorliegenden Erfindung Öle verstanden, welche bei 20 °C und einem Umgebungsdruck von 1.013 hPa einen Dampfdruck von weniger als 2,66 Pa (0,02 mm Hg) aufweisen.

Es kann erfindungsgemäß bevorzugt sein, Mischungen von flüchtigen Siliconölen und flüchtigen Nichtsiliconölen in den schweißhemmenden kosmetischen Mitteln einzusetzen, da hierdurch ein trockeneres Hautgefühl erreicht wird. Weiterhin kann es im Rahmen der vorliegenden Erfindung bevorzugt sein, wenn die schweißhemmenden kosmetischen Mittel ein nichtflüchtiges Siliconöl und/oder ein nichtflüchtiges Nichtsiliconöl enthalten, um unlösliche Bestandteile, wie Talkum oder auf der Haut angetrocknete Inhaltsstoffe, zu maskieren.

Erfindungsgemäß besonders bevorzugt ist der Einsatz von Mischungen von nichtflüchtigen und flüchtigen kosmetischen Ölen, da auf diese Weise Parameter wie Hautgefühl, Sichtbarkeit des Rückstands und Stabilität des schweißhemmenden kosmetischen Mittels eingestellt und das Mittel somit besser an die Bedürfnisse der Verbraucher angepasst werden kann.

Die im Rahmen der vorliegenden Erfindung einsetzbaren flüchtigen und nichtflüchtigen Siliconöle sowie flüchtigen und nichtflüchtigen Nichtsiliconöle sind beispielsweise in den Offenlegungsschriften DE 10 2010 063 250 A1 und DE 10 2012 222 692 A1 offenbart.

Gemäß einer bevorzugten Ausführungsform der vorliegenden Erfindung ist das bei 20 °C und 1.013 hPa flüssige kosmetische Öl in einer Gesamtmenge von 0,02 bis 98 Gew.-%, vorzugsweise von 2 bis 85 Gew.-%, bevorzugt von 4 bis 75 Gew.-%, weiter bevorzugt von 6 bis 70 Gew.-%, noch weiter bevorzugt von 8 bis 60 Gew.-%, insbesondere von 8 bis 20 Gew.-%, bezogen auf das Gesamtgewicht des schweißhemmenden kosmetischen Mittels, enthalten.

Als Bestandteil a) der kosmetischen Mittel kann ebenfalls mindestens ein Riechstoff enthalten sein. Bevorzugt werden jedoch Mischungen verschiedener Riechstoffe verwendet, welche gemeinsam eine ansprechende Duftnote erzeugen. Im Rahmen der vorliegenden Erfindung einsetzbare Riechstoffe sind beispielsweise in der Offenlegungsschrift DE 10 2010 063 250 A1 offenbart.

Besonders ansprechend riechende schweißhemmende kosmetische Mittel werden erhalten, wenn der mindestens eine Riechstoff in einer Gesamtmenge von 0,00001 bis 15 Gew.-%, vorzugsweise von 0,001 bis 9 Gew.-%, bevorzugt von 0,01 bis 8 Gew.-%, weiter bevorzugt von 0,1 bis 7 Gew.-%, noch weiter bevorzugt von 0,2 bis 6 Gew.-%, insbesondere von 0,2 bis 2 Gew.-%, bezogen auf das Gesamtgewicht des schweißhemmenden kosmetischen Mittels, enthalten ist.

Weiterhin können die kosmetischen Mittel als Bestandteil a) ein Wachs enthalten. Bevorzugt ist dieses Wachs ausgewählt aus der Gruppe von (i) Fettsäureglycerinmono-, -di- und -triestern; (ii) Butyrospermum Parkii (Shea Butter); (iii) Estern von gesättigten, einwertigen C₈₋₁₈-Alkoholen mit gesättigten C₁₂₋₁₈-Monocarbonsäuren; (iv) linearen, primären C_{12-C24}-Alkanolen; (v) Estern aus einem gesättigten, einwertigen C₁₆₋₆₀-Alkanol und einer gesättigten C_{8-C36}-Monocarbonsäure; (vi) Glycerintriestern von gesättigten linearen C₁₂₋₃₀-Garbonsäuren, die hydroxyliert sein können: (vii) natürlichen pflanzlichen Wachsen; (viii) tierischen Wachsen; (ix) synthetischen Wachsen; sowie (x) deren Mischungen. Im Rahmen der vorliegenden Erfindung bevorzugt einsetzbare Wachse sind in der Offenlegungsschrift DE 10 2012 222 692 A1 offenbart.

Im Rahmen der vorliegenden Erfindung ist es bevorzugt, wenn das Wachs in einer Gesamtmenge von 0,01 bis 50 Gew.-%, vorzugsweise von 3 bis 40 Gew.-%, bevorzugt von 5 bis 30 Gew-%, insbesondere von 6 bis 25 Gew.-%, bezogen auf das Gesamtgewicht des schweißhemmenden kosmetischen Mittels, enthalten ist.

Gemäß einer Ausführungsform der vorliegenden Erfindung kann es vorgesehen sein, dass die Milchproteine und/oder Joghurtproteine und/oder Seidenproteine in schweißhemmenden kosmetischen Mitteln verwendet werden, die als weiteren Bestandteil b) ein Treibmittel in einer Gesamtmenge von 0 bis 99 Gew.-%, bezogen auf das Gesamtgewicht des schweißhemmenden kosmetischen Mittels, enthalten. Wenn die kosmetischen Mittel ein Treibmittel enthalten, ist dieses bevorzugt in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%, bezogen auf das Gesamtgewicht des schweißhemmenden kosmetischen Mittels, enthalten. In diesem Fall sind die kosmetischen Mittel als treibgasgetriebene Aerosole konfektioniert. Bevorzugte Treibmittel (Treibgase) sind Propan, Propen, n-Butan, iso-Butan, isoButen, n-Pentan, Penten, iso-Pentan, iso-Penten, Methan, Ethan, Dimethylether, Stickstoff, Luft, Sauerstoff, Lachgas, 1,1,1,3-Tetrafluorethan, Heptafluoro-n-propan, Perfluorethan, Monochlordifluormethan, 1,1-Difluorethan, Tetrafluoropropene und zwar sowohl einzeln als auch in deren Mischungen. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Propan, n-Butan, iso-Butan sowie Mischungen dieser Treibgase. Es hat sich gezeigt, dass der Einsatz von n-Butan als einzigem Treibgas erfindungsgemäß besonders bevorzugt sein kann.

Eine besonders effektive Verminderung des Achselschweißes durch die Verwendung des mindestens eine Milchproteins und/oder Joghurtproteins und/oder Seidenproteins in schweißhemmenden kosmetischen Mitteln wird im Rahmen der vorliegenden Erfindung erreicht, wenn das mindestens eine Milchprotein und/oder Joghurtprotein und/oder Seidenprotein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%, bezogen auf das Gesamtgewicht des schweißhemmenden kosmetischen Mittels, enthalten ist. Ohne sich auf diese Theorie beschränken zu wollen resultiert der Einsatz der vorstehend genannten Mengen des mindestens einen Milchproteins und/oder Joghurtproteins und/oder Seidenproteins in einer signifikanten Beeinflussung der Schweißdrüse(n) durch Geldbildung des Proteins in den Ausführungsgängen der Schweißdrüsen oder durch Beeinflussung des Ladungsgleichgewichts innerhalb der Schweißdrüse(n). Auf diese Weise wird eine hervorragende schweißhemmende Wirkung gewährleistet. Weiterhin führt der Einsatz der zuvor genannten Mengen des mindestens einen Milchproteins und/oder Joghurtproteins und/oder Seidenproteins nicht zu instabilen Formulierungen, so dass die Stabilität der schweißhemmenden kosmetischen Mittel selbst über lange Lagerungszeiträume gewährleistet wird.

Besonders gute Ergebnisse im Hinblick auf die Verminderung und/oder Reduzierung der Achselnässe sowie auf die Hautverträglichkeit und die Lagerstabilität werden erhalten, wenn das mindestens eine Milchprotein und/oder Joghurtprotein und/oder Seidenprotein ein mittleres Molekulargewicht Mw von 150 bis 100,000 Da, vorzugsweise von 180 bis 50.000 Da, bevorzugt von 200 bis 10.000 Da, weiter bevorzugt von 250 bis 8.000 Da, insbesondere von 300 bis 5.000 Da, aufweist. Das mittlere Molekulargewicht Mw kann beispielsweise durch Gelpermeationschromatographie (GPC) bestimmt werden (Andrews P.; "Estimation of the Molecular Weights of Proteins by Sephadex Gel-Filtration"; Biochem. J., 1964, 91, Seiten 222 bis 233).

Gemäß einer bevorzugten Ausführungsform der vorliegenden Erfindung weist das mindestens eine Milchprotein und/oder Joghurtprotein und/oder Seidenprotein einen isoelektrischen Punkt auf, welcher im Bereich von pH 4,0 bis pH 10,0, vorzugsweise von pH 4,0 bis pH 9,5, insbesondere von pH 4,0 bis pH 8,0, liegt. Insbesondere Milchproteine und/oder Joghurtproteine und/oder Seidenproteine Proteine, welche einen isoelektrischen Punkt im vorgenannten pH-Bereich aufweisen, haben sich im Rahmen der vorliegenden Erfindung als vorteilhaft im Hinblick auf die schweißhemmende Wirkung sowie die Stabilität der kosmetischen Mittel erwiesen.

Eine besonders hohe schweißhemmende Wirkung, Hautverträglichkeit sowie Lagerstabilität wird im Rahmen der vorliegenden Erfindung gewährleistet, wenn das mindestens eine Milchprotein und/oder Joghurtprotein und/oder Seidenprotein eine Veränderung der Lichtabsorption bei einer pH-Wert Änderung von mindestens 0,5 in einem pH-Bereich von pH 4,5 bis pH 7,5, insbesondere von pH 5,0 bis pH 7,0, bei einer Konzentration von 0,001 bis 10 Gew.-% Milchprotein und/oder Joghurtprotein und/oder Seidenprotein, bezogen auf das Gesamtgewicht der zur pH-Messung verwendeten Probenmischung, und einer Temperatur von 20 °C bewirkt. Ohne sich auf diese Theorie beschränken zu wollen resultiert der Einsatz des mindestens einen Milchproteins und/oder Joghurtproteins und/oder Seidenproteins, welches in einem bestimmten pH-Bereich eine Veränderung der Lichtabsorption bewirkt, in einer signifikant erhöhten Beeinflussung der Schweißdrüse(n) durch pH-selektive Gelbildung in den Ausführungsgängen der Schweißdrüsen oder durch Störung des Ladungsgleichgewichts der Schweißdrüse(n), so dass eine hervorragende schweißhemmende Wirkung der kosmetischen Mittel gewährleistet wird, welche mit der schweißhemmenden Wirkung von aluminiumsalzhaltigen oder aluminium-zirkoniumsalzhaltigen kosmetischen Mitteln des Standes der Technik vergleichbar ist.

Im Rahmen der vorliegenden Erfindung ist es bevorzugt, wenn die pH-Wert Änderung durch Zugabe von Hydrogencarbonaten oder Carbonaten, insbesondere von Natriumhydrogencarbonaten, erreicht wird.

Gemäß einer bevorzugten Ausführungsform der vorliegenden Erfindung ist das mindestens eine Milchprotein und/oder Joghurtprotein und/oder Seidenprotein ausgewählt aus der Gruppe von (i) nicht modifizierten Proteinen; (ii) hydrolysierten Proteinen; (iii) chemisch modifizierten Proteinen, insbesondere hydrophob und/oder kationisch und/oder anionisch modifizierten Proteinen; (iv) physikalisch modifizierten Proteinen, insbesondere fraktionierten und/oder gereinigten und/oder bestrahlten Proteinen; (v) hydrolysierten nicht modifizierten Proteinen; (vi) hydrolysierten und chemisch modifizierten Proteinen, insbesondere hydrolysierten und hydrophob und/oder kationisch und/oder anionisch modifizierten Proteinen; (vii) hydrolysierten und physikalisch modifizierten Proteinen, insbesondere fraktionierten und/oder gereinigten und/oder bestrahlten Proteinen; sowie (viii) deren Mischungen.

Unter dem Begriff "nicht modifizierte Proteine" sind erfindungsgemäß Milchproteine und/oder Joghurtproteine und/oder Seidenproteine zu verstehen, welche weder mittels chemischer Verfahren, wie beispielsweise der Hydrolyse oder der chemischen Modifikation, noch mittels physikalischer Methoden, wie beispielsweise der Aufreinigung, der Trennung sowie der Bestrahlung, behandelt wurden.

Weiterhin sind unter dem Begriff "hydrolysierte Proteine" bzw. "Proteinhydrolysate" erfindungsgemäß Milchproteine und/oder Joghurtproteine und/oder Seidenproteine zu verstehen, welche durch chemische, insbesondere alkalische oder saure Hydrolyse, durch enzymatische Hydrolyse und/oder durch eine Kombination aus beiden Hydrolysearten hergestellt werden. Für den enzymatischen Abbau sind alle hydrolytisch wirkenden Enzyme geeignet, wie beispielsweise alkalische Proteasen. Übersichten zu Herstellung von Proteinhydrolysaten sind beispielsweise von G. Schuster und A. Domsch in Seifen Öle Fette Wachse 108, (1982) 177 bzw. Cosm. Toil. 99, (1984) 63, von H. W. Steisslinger in Parf.Kosm, 72, (1991) 556 und F. Aurich et al. in Tens.Surf.Det. 29, (1992) 389 erschienen. Mischungen von einzelnen Aminosäuren, welche lediglich durch Vermischen der Reinsubstanzen der Aminosäuren erhalten werden, sowie Totalhydrolysate, welche lediglich aus einzelnen Aminosäuren bestehen, fallen im Rahmen der vorliegenden Erfindung nicht unter den Begriff "hydrolysierte Proteine" bzw. "Proteinhydrolysate".

Darüber hinaus sind unter dem Begriff "chemisch modifizierte Proteine" im Rahmen der vorliegenden Erfindung Milchproteine und/oder Joghurtproteine und/oder Seidenproteine zu verstehen, welche durch chemische Umsetzung der reaktiven Gruppen der Proteine, insbesondere der Hydroxy-, Amin-, Imidazol-, Guanidino- und/oder Thiolgruppen der Seitenketten der Aminosäuren des Proteins, mit hydrophoben und/oder kationischen und/oder anionischen Verbindungen erhalten werden.

Zudem sind unter dem Begriff "physikalisch modifizierte Proteine" im Sinne der vorliegenden Erfindung Milchproteine und/oder Joghurtproteine und/oder Seidenproteine zu verstehen, welche durch physikalische Einwirkung, insbesondere durch Wärme und/oder Licht und/oder Fraktionierung, modifiziert wurden.

Im Rahmen dieser Ausführungsform ist es besonders bevorzugt, wenn das mindestens eine Milchprotein und/oder Joghurtprotein und/oder Seidenprotein ausgewählt ist aus der Gruppe von chemisch modifizierten, insbesondere hydrophob modifizierten, Proteinen. In diesem Zusammenhang weist das hydrophob modifizierte Milchprotein und/oder Joghurtproteinsund/oder Seidenprotein ein oder mehrere C₄₋₃₀-Kohlenstoffketten auf, wobei die C₄₋₃₀-Kohlenwasserstoffketten linear, cyclisch, verzweigt, unverzweigt, gesättigt, ungesättigt und aromatisch sein können und wobei die C₄₋₃₀-Kohlenwasserstoffketten über Ether-und/oder Ester- und/oder Amin-und/oder Amidbindungen an den Proteinrest gebunden sind.

Darüber hinaus ist es im Rahmen dieser Ausführungsform bevorzugt, wenn das mindestens eine Milchprotein und/oder Joghurtprotein und/oder Seidenprotein ausgewählt ist aus der Gruppe von chemisch modifizierten, insbesondere kationisch modifizierten, Proteinen. Bevorzugt enthält das kationisch modifizierte Milchprotein und/oder Joghurtprotein und/oder Seidenprotein daher einen oder mehrere Rest(e) der Formel R¹-N⁺(CH₃)₂-CH₂-CH(OH)-CH₂-X-R, in welcher R¹ für eine Alkylgruppe mit 1 bis 30 Kohlenstoffatomen, eine Alkenylgruppe mit 1 bis 30 Kohlenstoffatomen, eine Hydroxyalkylgruppe mit 1 bis 30 Kohlenstoffatomen, insbesondere für eine Methylgruppe, eine C₁₀₋₁₈-Alkyl oder eine C₁₀₋₁₆-Alkenylgruppe, X für O, N oder S und R für den Proteinrest stehen. Die Kationisierung der Milchproteine und/oder Joghurtproteine und/oder Seidenproteine mit den vorstehend beschriebenen Resten kann durch Umsetzung der Proteine mit den entsprechenden Halogeniden der obigen Formel erreicht werden, wobei die vorstehend beschriebenen Reste über Ether- und/oder Ester- und/oder Amid- und/oder Aminbindungen an das Protein gebunden sein können. Unter dem Begriff "Proteinrest" ist im Rahmen der vorliegenden Erfindung das durch die Verknüpfung von Aminosäuren gebildete Rückgrat des entsprechenden Proteins, an welches die kationische Gruppe über die zuvor genannten Bindungen gebunden ist, zu verstehen.

Es ist erfindungsgemäß weiterhin bevorzugt, wenn das mindestens eine Milchprotein und/oder Joghurtprotein und/oder Seidenprotein ein kationisch funktionalisiertes und/oder hydrolysiertes Protein ist.

Darüber hinaus ist es im Rahmen dieser Ausführungsform besonders bevorzugt, wenn das mindestens eine Milchprotein und/oder Joghurtprotein und/oder Seidenprotein eine oder mehrere Rest(e) der Formel R¹-N⁺(CH₃)₂-CH₂-CH(OH)-CH₂-X-R, in welcher R¹ für eine C₁₀₋₁₈-Alkyl- oder eine C₁₀₋₁₈-Alkenylgruppe, X für O, N oder S und R für den gegebenenfalls hydrolysierten Proteinrest stehen, enthält.

Ohne sich auf diese Theorie beschränken zu wollen resultiert der Einsatz dieser speziellen Milchproteine und/oder Joghurtproteine und/oder Seidenproteine in kosmetischen Mitteln in einer signifikant erhöhten Beeinflussung der Schweißdrüse(n) durch pH-selektive Gelbildung oder Störung des Ladungsgleichgewichts innerhalb der Ausführungsgänge der Schweißdrüsen. Auf diese Weise wird eine hervorragende schweißhemmende Wirkung der kosmetischen Mittel gewährleistet, welche mit der schweißhemmenden Wirkung von aluminiumsalzhaltigen oder aluminium-zirkoniumsalzhaltigen kosmetischen Mitteln des Standes der Technik vergleichbar ist. Darüber hinaus führt der Einsatz dieser speziellen Milchproteine und/oder Joghurtproteine und/oder Seidenproteine nicht zu einer negativen Wechselwirkung mit weiteren Inhaltsstoffen in schweißhemmenden kosmetischen Mitteln, so dass eine hohe Lagerstabilität der schweißhemmenden kosmetischen Mittel gewährleistet wird. Zudem weisen die kosmetischen Mittel eine hohe Hautverträglichkeit auf.

Es hat in diesem Zusammenhang als vorteilhaft herausgestellt, wenn das mindestens eine Milchprotein und/oder Joghurtprotein und/oder Seidenprotein Lysin in 1,0 bis 8,0 Mol.-%, Histidin in 1,0 bis 2,5 Mol.-% und Arginin in 2,0 bis 3,0 Mol,-%, bezogen auf die Gesamtstoffmenge aller Aminosäuren des Milchproteins und/oder Joghurtproteins und/oder Seidenproteins, enthält. Der Stoffmengenanteil der zuvor genannten Aminosäuren kann beispielsweise durch chromatographische Bestimmung unter Verwendung von sulfonierten Polystyrolharzen durchgeführt werden (Moore S. et. al; "Chromatography of amino acids on sulfonated polystyrene resin"; J. of Biological Chem., 1951, 192, Seiten 663 bis 681).

Weiterhin hat es sich in diesem Zusammenhang als vorteilhaft erwiesen, wenn das mindestens eine Milchprotein und/oder Joghurtprotein und/oder Seidenprotein einen Calciumgehalt von 1000 bis 15.000 mg, einen Kaliumgehalt von 400 bis 18,000 mg, einen Phosphorgehalt von 6.000 bis 11,000 mg und einen Schwefelgehalt von 3.000 bis 6.000 mg, jeweils bezogen auf 1 kg des mindestens einen Miichproteins und/oder Joghurtproteins und/oder Seidenproteins, aufweist. Die zuvor angegebenen Gehalte können beispielsweise mittels Atomemissionsspektrometrie (ICP-OES) nach Mikrowellenaufschluss mit Salpetersäure ermittelt werden (A. Oliveira et. al; "Evaluation of Metal Ions in Rice Samples: Extraction and Direct Determination by ICP-OES"; J. Braz. Chem. Soc., 2012, 23, Seiten 838 bis 845).

Gemäß einer weiteren besonders bevorzugten Ausführungsform der vorliegenden Erfindung bewirkt das mindestens eine Milchprotein und/oder Joghurtprotein und/oder Seidenprotein eine Veränderung der Lichtabsorption von 1,5 bis 90 %, vorzugsweise von 2 bis 80 %, bevorzugt von 2,5 bis 70 %, weiter bevorzugt von 3 bis 65 %, insbesondere von 3,5 bis 60 %. Die Veränderung der Lichtabsorption kann dabei auf einer Veränderung der Lichtdurchlässigkeit der Probenmischung, insbesondere durch Trübung, als auch auf die Absorption von Licht durch die Probenmischung, insbesondere durch das Protein selbst, erfolgen.

Die dieser Erfindung zugrundeliegenden Veränderungen der Lichtabsorption bei einer pH-Wert Änderung von mindestens 0,5 können durch Messung der Lichttransmission eines Lichtstrahls durch die Probenmischung bestimmt werden. Die Messungen der Lichttransmission wird unter Verwendung einer Methrom Optrode 6.1115.000 bei einer Wellenlänge von 574 nm (grüngelb) in mV (Auflösung 0,1 mV) in einem offenen Probengefäß bei 23 °C und 1.013 mbar durchgeführt. Die pH-Wert Änderung in dem pH-Bereich von 4,0 bis 8,0 wird durch langsame und kontinuierliche Zugabe einer Carbonat- oder Hydrogencarbonatlösung, bevorzugt einer 1 Gew.-%-igen Natriumhydrogencarbonatlösung, zu der Probenmischung unter Messung des pH-Werts mit einer pH-Elektrode sowie unter Rühren bei einer Geschwindigkeit von 750 bis 850 rpm erreicht. Die Veränderung der Lichtabsorption, welche durch das mindestens eine Protein bewirkt wird, berechnet sich nach der Formel ΔL = [(|L_{I}|/|L₀|]^{∗}100. In dieser Formel steht L_{I} für die Lichttransmission nach Veränderung des pH-Wertes um mindestens 0,5 in dem pH-Bereich von 4,0 bis 8,0, bevorzugt von pH 4,5 und 7,5, insbesondere von pH 5,0 und 7,0. L₀ steht in dieser Formel für die Differenz aus der Lichttransmission bei pH 4,0 und bei pH 8,0, bevorzugt bei pH 4,5 und bei pH 7,5, insbesondere bei pH 5,0 und bei pH 7,0, also beispielsweise Lichttransmission bei pH 8,0 minus Lichttransmission bei pH 4,0. Die Verwendung des mindestens einen speziellen Milchproteins und/oder Joghurtproteins und/oder Seidenproteins in schweißhemmenden kosmetischen Mitteln bewirkt eine nach obiger Methode bestimmte Veränderung der Lichtabsorption von 1 bis 100 %. Die vorliegende Erfindung ist jedoch nicht auf schweißhemmende kosmetische Zusammensetzungen beschränkt, welche mindestens ein spezielles Milchprotein und/oder Joghurtprotein und/oder Seidenprotein enthalten, welches eine nach obiger Methode bestimmte Veränderung der Lichtabsorption von 1 bis 100 % bewirkt. Sie umfasst auch die Verwendung von Milchproteinen und/oder Joghurtproteinen und/oder Seidenproteinen in schweißhemmenden kosmetischen Zusammensetzungen, welche nach anderen Methoden eine Veränderung der Lichtabsorption von 1 bis 100 % bewirken.

Im Rahmen der vorliegenden Erfindung ist es bevorzugt, wenn die Konzentration des mindestens einen Milchproteins und/oder Joghurtproteins und/oder Seidenproteins in der zur Bestimmung der Veränderung der Lichtabsorption verwendeten Mischung von 0,005 bis 10 Gew.-%, vorzugsweise von 0,05 bis 5 Gew.-%, bevorzugt von 0,07 bis 3 Gew.-%, insbesondere von 0,09 bis 2 Gew.-%, bezogen auf das Gesamtgewicht der für die Bestimmung der Veränderung der Lichtabsorption verwendeten Probenmischung, beträgt.

Erfindungsgemäß bevorzugt bewirkt das mindestens eine Milchprotein und/oder Joghurtprotein und/oder Seidenprotein eine Veränderung der Lichtabsorption bei einer pH-Wert Änderung von mindestens 0,5 und höchstens 3,5, vorzugsweise von mindestens 0,5 und höchstens 2,5, insbesondere von mindestens 0,5 und höchstens 1,5. Die Änderung des pH-Wertes kann insbesondere durch die Zugabe von Säuren oder Basen, bevorzugt Basen in Form von Carbonaten oder Hydrogencarbonaten, in der entsprechenden Menge erreicht werden.

Gemäß einer weiteren bevorzugten Ausführungsform der vorliegenden Erfindung weist das schweißhemmende kosmetische Mittel, in denen die Milchproteine und/oder Joghurtproteine und/oder Seidenproteine verwendet werden, einen pH-Wert von pH 2 bis pH 10 auf. Innerhalb dieses Bereichs ist eine stabile Formulierung der kosmetischen Mittel möglich, ohne dass unerwünschte Wechselwirkungen zwischen den Inhaltsstoffen der schweißhemmenden kosmetischen Mittel auftreten. Die Einstellung des gewünschten pH-Wertes kann erfindungsgemäß durch Verwendung von dem Fachmann bekannten und in schweißhemmenden kosmetischen Mitteln üblichen Säuren und Basen erfolgen.

Erfindungsgemäß ist es weiterhin bevorzugt, wenn das schweißhemmende kosmetische Mittel zusätzlich mindestens ein Konservierungsmittel enthält. Erfindungsgemäß bevorzugte Konservierungsmittel sind Formaldehydabspalter Iodopropinylbutylcarbamate, Parabene, Phenoxyethanol, Ethanol, Benzoesäure und deren Salze, Dibromdicyanobutan, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol, Salicylsäure und Salicylate. Weitere im Rahmen der vorliegenden Erfindung einsetzbare Konservierungsmittel sind die in Anlage 6 der Kosmetikverordnung aufgeführten Substanzen sowie kosmetische Rohstoffe mit konservierenden Eigenschaften oder Rohstoffe, welche die konservierende Wirkung der vorgenannten Konservierungsmittel unterstützen bzw. verstärken. Die Konservierungsmittel sind vorzugsweise in einer Gesamtmenge von 0,01 bis 10 Gew.-%, vorzugsweise von 0,1 bis 7 Gew.-%, bevorzugt von 0,2 bis 5 Gew.-%, insbesondere von 0,3 bis 2,0 Gew.-%, bezogen auf das Gesamtgewicht des schweißhemmenden kosmetischen Mittels, enthalten.

Im Rahmen der vorliegenden Erfindung ist es bevorzugt, wenn das schweißhemmende kosmetische Mittel als Wasser-in-ÖI-Emulsion vorliegt. Hierbei kann es sich insbesondere um eine versprühbare Wasser-in-Öl-Emulsion handeln, welche mittels eines Treibmittels versprüht werden kann. In diesem Zusammenhang ist es bevorzugt, wenn das in Form einer Wasser-in-Öl-Emulsion vorliegende schweißhemmende kosmetische Mittel das mindestens eine Milchprotein und/oder Joghurtprotein und/oder Seidenprotein in einer Gesamtmenge von 0,1 bis 70 Gew.-%, vorzugsweise von 0,5 bis 60 Gew.-%, bevorzugt von 1,0 bis 50 Gew.-%, weiter bevorzugt von 1,5 bis 40 Gew-%, noch weiter bevorzugt von 2,0 bis 30 Gew.-%, insbesondere von 2,0 bis 20 Gew.-%, bezogen auf das Gesamtgewicht des schweißhemmenden kosmetischen Mittels, enthält.

Es kann jedoch erfindungsgemäß gleichermaßen bevorzugt sein, wenn das schweißhemmende kosmetische Mittel als Öl-in-Wasser-Emulsion vorliegt. In diesem Fall wird das kosmetische Mittel bevorzugt als treibmittelfreies Pumpspray oder Quetschspray versprüht oder als Roll-on appliziert. In diesem Zusammenhang ist es bevorzugt, wenn das in Form einer Öl-in-Wasser-Emulsion vorliegende schweißhemmende kosmetische Mittel das mindestens eine Milchprotein und/oder Joghurtprotein und/oder Seidenprotein in einer Gesamtmenge von 0,1 bis 70 Gew.-%, vorzugsweise von 0,5 bis 60 Gew.-%, bevorzugt von 1,0 bis 50 Gew.-%, weiter bevorzugt von 1,5 bis 40 Gew-%, noch weiter bevorzugt von 2,0 bis 30 Gew.-%, insbesondere von 2,0 bis 20 Gew.-%, bezogen auf das Gesamtgewicht des schweißhemmenden kosmetischen Mittels, enthält.

Gemäß einer weiteren bevorzugten Ausführungsform der vorliegenden Erfindung können die kosmetischen Mittel nur einen geringen Gehalt an freiem Wasser bzw. kein freies Wasser enthalten. Unter freiem Wasser im Sinne der vorliegenden Erfindung wird Wasser verstanden, welches von Kristallwasser, Hydratationswasser oder ähnlich molekular gebundenem Wasser der eingesetzten Bestandteile verschieden ist. Das schweißhemmende kosmetische Mittel enthält bevorzugt freies Wasser in einer Gesamtmenge von weniger als 10 Gew.-%, vorzugweise von weniger als 8 Gew.-%, bevorzugt von weniger als 5 Gew.-%, weiter bevorzugt von weniger als 3 Gew.-%, noch mehr bevorzugt von weniger als 1 Gew.-%, insbesondere von 0 Gew.-%, bezogen auf das Gesamtgewicht des schweißhemmenden kosmetischen Mittels.

Im Rahmen einer weiteren Ausführungsform ist es jedoch erfindungsgemäß ebenfalls bevorzugt, wenn das schweißhemmende kosmetische Mittel als wässrige, wässrig-alkoholische oder wässrig-glykolische Lösung vorliegt. Da die kosmetischen Mittel, in denen mindestens ein Milchprotein und/oder Joghurtprotein und/oder Seidenprotein verwendet wird, keine schweißhemmenden Halogenide und/oder Hydroxyhalogenide von Aluminium und/oder Zirconium enthalten, welche durch den Zusatz protischer Lösungsmittel eine verminderte schweißhemmende Wirkung aufweisen, können erfindungsgemäß protische Lösungsmittel, wie wässrige Lösungen, zur Formulierung der schweißhemmenden kosmetischen Mittel verwendet werden, ohne dass eine signifikante Verringerung der Antitranspirantwirkung auftritt. Daher gewährleistet der Zusatz des mindestens einen speziellen Milchproteins und/oder Joghurtproteins und/oder Seidenproteins selbst bei Verwendung von protischen Lösungsmitteln eine effektive Beeinflussung der der Schweißdrüse(n) und somit eine hervorragende Antitranspirantwirkung.

Im Zusammenhang mit dieser Ausführungsform der vorliegenden Erfindung wurde Überraschenderweise festgestellt, dass die Beeinflussung der Schweißdrüse(n) durch das mindestens eine spezielle Milchprotein und/oder Joghurtprotein und/oder Seidenprotein signifikant gesteigert werden kann, wenn die schweißhemmenden kosmetischen Mittel freies Wasser in einer Menge von 5 bis 99 Gew.-%, bezogen auf das Gesamtgewicht des schweißhemmenden kosmetischen Mittels, enthalten. In einer besonders bevorzugten Ausführungsform der vorliegenden Erfindung enthält das schweißhemmende kosmetische Mittel daher freies Wasser in einer Gesamtmenge von 5 bis 96 Gew.-%, vorzugsweise von 15 bis 80 Gew.-%, bevorzugt von 30 bis 70 Gew.-%, insbesondere von 40 bis 60 Gew.-%, bezogen auf das Gesamtgewicht des schweißhemmenden kosmetischen Mittels.

Weiterhin ist es im Zusammenhang mit dieser Ausführungsform bevorzugt, wenn das schweißhemmende kosmetische Mittel Ethanol in einer Gesamtmenge von 1 bis 99 Gew.-%, vorzugsweise von 5 bis 70 Gew.-%, bevorzugt von 7 bis 50 Gew.-%, insbesondere von 10 bis 30 Gew.-%, bezogen auf das Gesamtgewicht des schweißhemmenden kosmetischen Mittels, enthält. Wie zuvor ausgeführt, können durch den Einsatz des mindestens einen speziellen Milchproteins und/oder Joghurtproteins und/oder Seidenproteins selbst hohe Mengen an protischen Lösungsmitteln, wie Ethanol, verwendet werden, ohne dass die Antitranspirantwirkung des erfindungsgemäßen schweißhemmenden kosmetischen Mittels negativ beeinflusst wird.

Die Applikation des schweißhemmenden kosmetischen Mittels kann mittels verschiedener Verfahren erfolgen. Gemäß einer bevorzugten Ausführungsform ist das schweißhemmende kosmetische Mittel als Spray-Applikation konfektioniert. Die Spray-Applikation erfolgt mit einer Sprühvorrichtung, welche in einem Behälter eine Füllung aus dem erfindungsgemäßen flüssigen, viskos-fließfähigen, suspensionsförmigen oder pulverförmigen schweißhemmenden kosmetischen Mittel enthält Die Füllung kann unter dem Druck eines Treibmittels stehen (Druckgasdosen, Druckgaspackungen, Aerosolpackungen), oder es kann sich um einen mechanisch zu bedienenden Pumpzerstäuber ohne Treibgas (Pumpsprays/ Quetschflasche) handeln. Die Zerstäubung des schweißhemmenden kosmetischen Mittels kann hierbei physikalisch, mechanisch oder elektromechanisch, beispielsweise durch Piezzoeffekte oder elektrische Pumpen, erfolgen. Im Rahmen dieser Ausführungsform einsetzbare Behälter und Entnahmevorrichtungen sind beispielsweise in der Offenlegungsschrift DE 10 2012 222 692 A1 beschrieben.

Das schweißhemmende kosmetische Mittel kann weiterhin bevorzugt als Stift, Soft Solid, Creme, Gel, Roll-on, loses oder kompaktes Puder konfektioniert sein. Die Formulierung der schweißhemmenden kosmetischen Mittel in einer bestimmten Darreichungsform, wie beispielsweise einem Antitranspirant-Roll-on, einem Antitranspirantstift oder einem Antitranspirantgel, richtet sich bevorzugt nach den Anforderungen des Verwendungszwecks. Je nach Verwendungszweck können die schweißhemmenden kosmetischen Mittel daher in fester, halbfester, flüssiger, disperser, emulgierter, suspendierter, gelförmiger, mehrphasiger oder puderförmiger Form vorliegen. Unter den Begriff der Flüssigkeit fallen im Sinne der vorliegenden Erfindung auch jegliche Arten von Festkörperdispersionen in Flüssigkeiten.

Weiterhin werden unter mehrphasigen schweißhemmenden kosmetischen Mitteln im Sinne der vorliegenden Erfindung Mittel verstanden, welche mindestens 2 verschiedene Phasen mit einer Phasentrennung aufweisen und bei welchen die Phasen horizontal, also übereinander, oder vertikal, also nebeneinander, angeordnet sein können. Die Applikation kann beispielsweise mit einem Rollkugelapplikator oder mittels eines festen Stifts erfolgen.

Es kann im Rahmen der vorliegenden Erfindung ebenfalls bevorzugt sein, wenn das schweißhemmende kosmetische Mittel auf und/oder in einem wegwerfbaren Substrat, ausgewählt aus der Gruppe von Tüchern, Pads und Bauschen, enthalten ist. Besonders bevorzugt sind Feuchttücher, d.h. für den Anwender vorgefertigte, bevorzugt einzeln abgepackte, Feuchttücher, wie sie z. B. aus dem Bereich der Glasreinigung oder aus dem Bereich der feuchten Toilettenpapiere wohlbekannt sind. Solche Feuchttücher, die vorteilhafter Weise auch Konservierungsstoffe enthalten können, sind mit einem chweißhemmenden kosmetischen Mittel imprägniert oder beaufschlagt und bevorzugt einzeln verpackt. Bevorzugte Substratmaterialien sind ausgewählt aus porösen flächigen Tüchern. Zu diesen Tüchern gehören Tücher aus gewebten und ungewebten (Vlies) synthetischen und natürlichen Fasern, Filz, Papier oder Schaumstoff, wie hydrophilem Polyurethanschaum. Erfindungsgemäß bevorzugte deodorierende oder schweißhemmende Substrate können durch Tränken oder Imprägnierung oder auch durch Aufschmelzen eines schweißhemmenden kosmetischen Mittels auf ein Substrat erhalten werden.

Erfindungsgemäß bevorzugt enthält das schweißhemmende kosmetische Mittel mindestens einen weiten Hilfsstoff, ausgewählt aus der Gruppe von (i) Emulgatoren und/oder Tensiden; (ii) Verdickungsmitteln; (iii) Chelatbildnern; (iv) Deodorant-Wirkstoffen; (v) ein- und/oder mehrwertigen Alkoholen und/oder Polyethylenglycolen; (vi) hautkühlenden Wirkstoffen; (vii) pH-Stellmitteln; (viii) hautpflegenden Wirkstoffen, wie Moisturizern, hautberuhigenden Stoffen, hautaufhellenden Stoffen, hautglättenden Stoffen; sowie (ix) deren Mischungen.

Erfindungsgemäß bevorzugt geeignete Emulgatoren und Tenside sind ausgewählt aus anionischen, kationischen, nichtionischen, amphoteren, insbesondere ampholytischen und zwitterionischen Emulgatoren und Tensiden. Tenside sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8 bis 28 Kohlenstoffatomen, die gesättigt oder ungesättigt, linear oder verzweigt sein kann. Besonders bevorzugt ist diese C₈-C₂₈-Alkylkette linear. Im Rahmen der vorliegenden Erfindung bevorzugt einsetzbare Emulgatoren und Tenside sind beispielsweise in den Offenlegungsschriften DE 10 2012 222 692 A1, DE 10 2010 063 250 A1 sowie DE 10 2010 055 816 A1 offenbart.

Zur Verdickung der schweißhemmenden kosmetischen Mittel werden bevorzugt Substanzen eingesetzt, welche ausgewählt sind aus Celluloseethern, Xanthan-Gum, Sclerotium Gum, Succinoglucanen, Polygalactomannanen, Pectinen, Agar, Carragheen (Carrageenan), Traganth, Gummi arabicum, Karayagummi, Taragummi, Gellan, Gelatine, Propylenglycolalginat, Alginsäuren und deren Salze, Polyvinylpyrrolidonen, Polyvinylalkoholen, Polyacrylamiden, physikalisch (z. B. durch Vorverkleisterung) und/oder chemisch modifizierten Stärken, Acrylsäure-Acrylat-Copolymeren, Acrylsäure-Acrylamid-Copolymeren, Acrylsäure-Vinylpyrrolidon-Copolymeren, Acrylsäure-Vinyiformamid-Copolymeren und Polyacrylaten. Besonders bevorzugte Verdickungsmittel sind weiterhin ausgewählt aus Carbomeren. Carbomere sind verdickende vernetzte Polymere aus Acrylsäure, Methacrylsäure sowie deren Salzen. Die Vernetzung kann mittels polyfunktioneller Verbindungen wie Polyalkylenether von Polysacchariden oder Polyalkoholen, beispielsweise Sucroseallylether, Pentaerythritolallylether, Propylenallylether, erfolgen. Bevorzugt im Rahmen der vorliegenden Erfindung sind Homopolymere von Acrylsäure oder deren Salzen, welche mit einem Pentaerythritolallylether, einem Sucroseallylether oder einem Propylenallylether vernetzt sind. Ein im Rahmen der vorliegenden Erfindung einsetzbares Verdickungsmittel ist ein Copolymer aus C₁₀₋₃₀-Alkylacrylat, Acrylsäure, Methacrylsäure sowie deren Estern, welches mit einem Sucroseallylether oder einem Pentaerythritolallylether vernetzt ist. Verdickungsmittel auf Basis von Carbomeren sind die unter dem Handelsnamen Carbopol® (BF Goodrich, Ohio, USA) erhältlichen Produkte wie beispielsweise Carbopol 934, Carbopol 940, Carbopol 941, Carbopol 971, Carbopol 974, Carbopol EZ2, Carbopol ETD 2001, Carbopol ETD 2020, Carbopol ETD 2050, Carbopol ultrez 10, Carbopol ultrez 20, oder Carbopol ultrez 21.

Weiterhin können zur Verdickung der schweißhemmenden kosmetischen Mittel lipophile Verdickungsmittel eingesetzt werden. Erfindungsgemäß bevorzugte lipophile Verdickungsmittel sind ausgewählt aus hydrophobierten Tonmineralien, Bentoniten, pyrogenen Kieselsäuren sowie deren Derivaten.

Um die Beeinflussung der Schweißdrüse(n) durch das mindestens eine spezielle Milchprotein und/oder Joghurtprotein und/oder Seidenprotein weiter zu unterstützen, kann es von Vorteil sein, den schweißhemmenden kosmetischen Mitteln mindestens einen Chelatbildner, in einer Gesamtmenge von 0,01 bis 3,0 Gew.-%, vorzugsweise von 0,02 bis 1,0 Gew.-%, insbesondere von 0,05 bis 0,1 Gew.-%, bezogen auf das Gesamtgewicht des schweißhemmenden Mittels, zuzusetzen. Im Rahmen der vorliegenden Erfindung sind bevorzugte Chelatbildner ausgewählt aus der Gruppe von β-Alanindiessigsäure, Cyclodextrin, Diethylentriaminpentamethylenphosphonsäure, Natrium-, Kalium-, Calciumdinatrium-, Ammonium- und Triethanolaminsalzen der Ethylendiamintetraessigsäure (EDTA), Etidronsäure, Hydroxyethylethylendiamintetraessigsäure (HEDTA) und ihren Natriumsalzen, Natriumsalzen der Nitrilotriessigsäure (NTA), Diethylentriaminpentaessigsäure, Phytinsäure, Hydroxypropylcyclodextrin, Methylcyclodextrin, Aminotrimethylenphosphonat-Pentanatriurn, Ethylendiamintetramethylenphosphonat-Pentanatrium, Diethylentriamin-pentaacetat-Pentanatrium, Pentanatriumtriphosphat, Kalium-EDTMP, Natrium-EDTMP, Natriumdihydroxyethylglycinat, Natriumphytat, Natriumpolydimethyigiycinophenolsulfonat, Tetrahydroxyethylethylendiamin, Tetrahydroxypropylethylendiamin, Tetrakaliumetidronat, Tetranatriumetidronat, Tetranatriumiminodisuccinat, Trinatriumethylendiamindisuccinat, Tetranatrium-N,N-bis(Carboxymethyl)glutamat, Tetranatrium-DL-Alanin-N,N-diacetat und Desferrioxamin.

Die desodorierende Wirkung der schweißhemmenden kosmetischen Mittel kann weitergehend gesteigert werden, wenn mindestens ein Deodorant-Wirkstoff mit antibakterieller und/oder bakteriostatischer und/oder enzyminhibierender und/oder geruchsneutralisierender und/oder geruchsabsorbierender Wirkung in einer Gesamtmenge von 0,0001 bis 40 Gew.-%, vorzugsweise von 0,2 bis 20 Gew.-%, bevorzugt von 1 bis 15 Gew.-%, insbesondere von 1,5 bis 5 Gew.-%, bezogen auf das Gesamtgewicht des schweißhemmenden kosmetischen Mittels, enthalten ist. Sofern Ethanol in den Mitteln eingesetzt wird, gilt dieses im Rahmen der vorliegenden Erfindung nicht als Deodorant-Wirkstoff, sondern als Bestandteil des Trägers. Erfindungsgemäß bevorzugte Deodorant-Wirkstoffe sind beispielsweise in der Offenlegungsschrift DE 10 2010 063 250 A1 offenbart.

Bevorzugte schweißhemmende kosmetische Mittel enthalten weiterhin mindestens ein wasserlösliches mehrwertiges C₂₋₉-Alkanol mit 2 bis 6 Hydroxylgruppen und/oder mindestens ein wasserlösliches Polyethylenglycol mit 3 bis 50 Ethylenoxid-Einheiten sowie Mischungen hiervon. Hierunter fallen nicht die vorstehend erwähnten Deodorant-Wirkstoffe in Form von 1,2-Alkandiolen. Bevorzugte Alkanole und wasserlösliche Polyethylenglycole sind beispielsweise in der Offenlegungsschrift DE 10 2010 063 250 A1 beschrieben.

Gemäß einer weiteren Ausführungsform der vorliegenden Erfindung enthalten die schweißhemmenden kosmetischen Mittel weiterhin mindestens einen hautkühlenden Wirkstoff. Erfindungsgemäß geeignete hautkühlende Wirkstoffe sind beispielsweise Menthol, Isopulegol sowie Mentholderivate, z. B. Menthyllactat, Menthylglycolat, Menthyl Ethyl Oxamate, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat, 2-Hydroxymethyl-3,5,5-trimethylcyclohexanol und 5-Methyl-2-(1-methylethyl)cyclohexyl-N-ethyloxamat. Als hautkühlende Wirkstoffe bevorzugt sind Menthol, Isopulegol, Menthyllactat, Menthoxypropandiol, Menthylpyrrolidoncarbonsäure und 5-Methyl-2-(1-methylethyl)cyclohexyl-N-ethyloxamat sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.

Als pH-Stellmittel kommen erfindungsgemäß bevorzugt Säuren und/oder Alkalisierungsmittel und/oder Puffer zum Einsatz. Als Säuren werden erfindungsgemäß bevorzugt anorganische Säuren (wie beispielsweise Salzsäure, Schwefelsäure oder Phosphorsäure) oder organische Säuren (wie beispielsweise Zitronensäure, Weinsäure oder Apfelsäure) eingesetzt. Die erfindungsgemäß verwendbaren Alkalisierungsmittel werden bevorzugt ausgewählt aus der Gruppe, welche gebildet wird, aus Ammoniak, basischen Aminosäuren, Alkalihydroxiden, Carbonaten und Hydrogencarbonaten, Alkanolaminen, beispielsweise Amino-2-methyl-1-propanol, Monoethanolamin, Triethanolamin, Diethanolamin und Triisopropanolamin, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium. Als Puffersysteme eignen sich im Rahmen der vorliegenden Erfindung insbesondere Kohlensäure-Bicarbonat-Puffer, Kohlensäure-Silicat-Puffer, Essigsäure-Acetat-Puffer, Phosphatpuffer, Ammoniakpuffer, Citronensäure- oder Citratpuffer, Puffer auf Basis von Tris(hydroxymethyl)-aminomethan, Puffer auf Basis von 4-(2-Hydroxyethyl)-1-piperazinethanesulfonsäure, Puffer auf Basis von 4-(2-Hydroxyethyl)-piperazin-1-propansulfonsäure, Puffer auf Basis von 2-(N-Morpholino)ethansulfonsäure sowie Barbital-Acetat-Puffer. Die Wahl des entsprechenden Puffersystems richtet sich hierbei nach dem gewünschten pH-Wert der schweißhemmenden kosmetischen Mittel.

In einer bevorzugten Ausführungsform sind die schweißhemmenden kosmetischen Mittel, in denen mindestens ein Milchprotein und/oder Joghurtprotein und/oder Seidenprotein verwendet wird, dadurch gekennzeichnet, dass sie - bezogen auf das Gesamtgewicht des schweißhemmenden kosmetischen Mittels -
- mindestens ein Milchprotein und/oder Joghurtprotein und/oder Seidenprotein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Milchprotein und/oder Joghurtprotein und/oder Seidenprotein bei einer pH-Wert Änderung von mindestens 0,5 in einem pH-Bereich von pH 4,0 bis pH 8,0, einer Temperatur von 20 °C bis 40 °C und einer Konzentration des Milchproteins und/oder Joghurtproteins und/oder Seidenproteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer weiteren bevorzugten Ausführungsform sind die schweißhemmenden kosmetischen Mittel in denen mindestens ein Milchprotein und/oder Joghurtprotein und/oder Seidenprotein verwendet wird, dadurch gekennzeichnet, dass sie - bezogen auf das Gesamtgewicht des schweißhemmenden kosmetischen Mittels -
- mindestens ein Milchprotein und/oder Joghurtprotein und/oder Seidenprotein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens einen Emulgator und/oder ein Tensid,
- mindestens ein pH-Stellmittel,
- mindestens ein Konservierungsmittel,
- 0,01 bis 2 Gew.-%, vorzugsweise 0,1 bis 1 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Milchprotein und/oder Joghurtprotein und/oder Seidenprotein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer bevorzugten Ausführungsform sind die schweißhemmenden kosmetischen Mittel in denen mindestens ein Milchprotein und/oder Joghurtprotein und/oder Seidenprotein verwendet wird, dadurch gekennzeichnet, dass sie - bezogen auf das Gesamtgewicht des schweißhemmenden kosmetischen Mittels -
- mindestens ein Milchprotein und/oder Joghurtprotein und/oder Seidenprotein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew,-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel und
- mindestens einen Stoff, ausgewählt aus der Gruppe von kosmetischen Ölen, welche bei 20 °C und 1,013 hPa flüssig sind, Riechstoffen und Wachsen
enthalten, wobei das mindestens eine Milchprotein und/oder Joghurtprotein und/oder Seidenprotein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

In einer weiteren bevorzugten Ausführungsform sind die schweißhemmenden kosmetischen Mittel in denen mindestens ein Milchprotein und/oder Joghurtprotein und/oder Seidenprotein verwendet wird, dadurch gekennzeichnet, dass sie - bezogen auf das Gesamtgewicht des schweißhemmenden kosmetischen Mittels -
- mindestens ein Milchprotein und/oder Joghurtprotein und/oder Seidenprotein in einer Gesamtmenge von 0,5 bis 60 Gew.-%, vorzugsweise von 1,0 bis 50 Gew.-%, bevorzugt von 1,5 bis 40 Gew-%, weiter bevorzugt von 2,0 bis 30 Gew.-%, insbesondere von 2,0 bis 20 Gew.-%,
- 12 bis 98 Gew.-%, vorzugsweise 25 bis 55 Gew.-%, bevorzugt 30 bis 50 Gew.-%, insbesondere 35 bis 45 Gew.-% Wasser,
- mindestens ein Treibmittel in einer Gesamtmenge von 1 bis 98 Gew.-%, vorzugsweise von 20 bis 90 Gew.-%, bevorzugt von 30 bis 85 Gew-%, insbesondere von 40 bis 75 Gew.-%,
- mindestens einen Emulgator und/oder ein Tensid,
- mindestens ein pH-Stellmittel,
- mindestens ein Konservierungsmittel,
- 0,01 bis 2 Gew.-%, vorzugsweise 0,1 bis 1 Gew.-%, bevorzugt 0,2 bis 0,7 Gew.-%, insbesondere 0,3 bis 0,5 Gew.-% eines Verdickungsmittels, und
- mindestens einen Stoff, ausgewählt aus der Gruppe von kosmetischen Ölen, welche bei 20 °C und 1.013 hPa flüssig sind, Riechstoffen und Wachsen
enthalten, wobei das mindestens eine Milchprotein und/oder Joghurtprotein und/oder Seidenprotein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt.

Im Rahmen der vorliegenden Erfindung kann es auch vorgesehen sein, das kosmetische Mittel als ZweiKomponenten-Mittel zu konfektionieren. Die einzelnen Komponenten werden hierzu vorzugsweise in getrennten Containern gelagert und in beliebiger Reihenfolge nacheinander oder gleichzeitig auf die Haut aufgetragen. Eine Auftrennung in Mehrkomponentensysteme ist insbesondere dort bevorzugt, wo Inkompatibilitäten der Inhaltsstoffe zu erwarten oder zu befürchten sind.

Darüber hinaus ist ein weiterer Gegenstand der vorliegenden Erfindung die Verwendung einer Kombination, enthaltend
a) mindestens einen Stoff, ausgewählt aus der Gruppe von kosmetischen Ölen, welche bei 20 °C und 1.013 hPa flüssig sind, Riechstoffen und Wachsen,
b) Treibmittel in einer Gesamtmenge von 0 bis 99 Gew.-%, bezogen auf das Gesamtgewicht des schweißhemmenden kosmetischen Mittels, und
c) mindestens ein Milchprotein und/oder Joghurtprotein und/oder Seidenprotein in einer Gesamtmenge von 0,1 bis 70 Gew.-%, bezogen auf das Gesamtgewicht des schweißhemmenden kosmetischen Mittels, wobei das mindestens eine Protein in tierischen Sekreten, Sekreten von Insekten oder humanen Sekreten vorkommt, wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption von 1 bis 100 %, bewirkt und
wobei die Kombination keine Halogenide und/oder Hydroxyhalogenide von Aluminium und/oder Zirconium enthält,
zur Reduzierung und/oder Verhinderung von Schweiß, insbesondere Achselschweiß oder Schweiß anderer Körperregionen.

Der Begriff der "Kombination" im Sinne der vorliegenden Erfindung umfasst eine Mischung der oben angegebenen Inhaltsstoffe a), b) und c). Bezüglich der Verwendung der vorstehend genannten Kombination gilt mutatis mutandis das zu der erfindungsgemäßen Verwendung Gesagte.

Die folgenden Beispiele erläutern die vorliegende Erfindung, ohne sie jedoch darauf einzuschränken:

### Beispiele:

### 1. Veränderung der Lichtabsorption

Es wurden folgende Proteine verwendet:
1) Hydrolysiertes Milchprotein mit mindestens einer Lauryldimoniumhydroxypropylgruppe und einem mittleren Molekulargewicht Mw von etwa 1.300 Da und
2) Hydrolysiertes Casein mit einem mittleren Molekulargewicht Mw von etwa 600 Da und
3) Hydrolysiertes Milchprotein mit einem mittleren Molekulargewicht Mw von etwa 1.800 Da und
4) Sprühgetrocknetes Joghurtprotein aus fettarmem Joghurt mit einem mittleren Molekulargewicht Mw von 3.000 bis 35.000 Da.

Die Bestimmung der Veränderung der Lichtabsorption, welche durch die zuvor genannten Proteine in einem pH-Bereich von 4,0 bis 8,0 bei einer pH-Wert Änderung von mindestens 0,5 bewirkt wird, wird folgendermaßen durchgeführt:

**Tabelle 1: Probenlösung (Angaben in Gew.-%)**

| | E-I* | E-II* | EIII* | E-IV* | |
|---|---|---|---|---|---|
| Hydrolysiertes Milchprotein mit mindestens einer Lauryldimoniumhydroxypropylpruppe ^{a)} | 0,5 | -- | -- | -- | |
| Hydrolysiertes Casein ^{b)} | -- | 3,3 | -- | -- | |
| Hydrolysiertes Michprotein ^{c)} | -- | -- | 1,1 | -- | |
| Joghurtprotein ^{d)} | -- | -- | -- | 0,29 | |
| HCl | Add pH | Add pH | Add pH | Add pH | |
| Wasser | Add 100 | Add 100 | Add 100 | Add 100 | |

| | | | | | |
|---|---|---|---|---|---|
| * erfindungsgemäß ^{a)} Hydrolactin QM LQ (WD); (INCI: Cocodimonium Hydroxypropyl Hydrolyzed Milk Protein; 20 Gew.-%ige Lösung in Wasser; M_{w} etwa 1.300 Da; Croda) ^{b)} Promois Milk; (INCI: Hydrolyzed Casein: 30 Gew.-%ige Lösung in Wasser; M_{w} etwa 600 Da; Seiwa Kasai CO. LTD) ^{c)} Hydrolactin 2500 PW (WD); (INCI: Hydrolyzed Milk Protein; 90% Gew.-%ige Lösung in Wasser; M_{w} etwa 1.800 Da; Croda) ^{d)} Yoghurt Protein GBU (INCI: Yoghurt; 35 Gew.-%ige Lösung in Wasser; M_{w} etwa 3.000 bis 35.000 Da; Cosmetochem) | | | | | |

Für die Bestimmung der Veränderung der Lichtabsorption wurde ein Methrom Titrando 905 der Firma Methrom (USA) verwendet, welches mit einer Methrom Optrode 6.1115.000 sowie einer pH-Elektrode von Methrom ausgestattet ist. Die Steuerung des Methrom Titrando 905 erfolgt über die Software Tiamo der Firma Methrom, Zunächst wurden 30 ml einer Probenlösung gemäß Tabelle 1, welche einen pH-Wert von 3,0 aufwies, in dem offenen Probengefäß des Methrom Titrando 905 vorgelegt. Anschließend wurde bei 23 °C und 1.013 mbar unter Rühren (Rührgeschwindigkeit 8 des Titrando 905, entspricht ca. 750 bis 850 rpm) solange kontinuierlich eine 1 Gew.-%-ige Natriumhydrogencarbonatlösung zugegeben, bis ein pH-Wert von 7,5 erreicht wurde. Während der Zugabe der 1 Gew,-%-igen Natriumhydrogencarbonatlösung wurde die Lichttransmission eines Lichtstrahls durch diese Probenlösung mit einer Methrom Optrode 6.1115.000 bei einer Wellenlänge von 574 nm (grüngelb) in mV (Auflösung 0,1 mV) gemessen. Jede Messung wird jeweils zweimal durchgeführt und hieraus der Mittelwert gebildet.

Die Veränderung der Lichtabsorption, welche durch die oben genannten Peptide bewirkt wurde, wurde gemäß der Formel ΔL = [(|L_{I}|/|L₀|]^{∗}100 bestimmt. In dieser Formel steht Lᵢ für die Lichttransmission nach Veränderung des pH-Wertes um mindestens 0,5 in dem pH-Bereich von 4,0 bis 8,0, bevorzugt von pH 4,5 und 7,5, insbesondere von pH 5,0 und 7,0. L₀ steht in dieser Formel für die Differenz aus der Lichttransmission bei pH 4,0 und bei pH 8,0, bevorzugt bei pH 4,5 und bei pH 7,5, insbesondere bei pH 5,0 und bei pH 7,0.

Bei einer pH-Wert Änderung von 1,0 zwischen pH 5,0 und pH 6,0 (Lichtabsorption bei pH 6,0 bildet Wert Lᵢ) in einem pH-Wertbereich von 4,5 bis 7,5 (Differenz Lichtabsorption bei pH 7,5 minus pH 4,5 bildet Wert L₀) bewirkten diese Proteine die in Tabelle 2 angegebene Veränderung der Lichtabsorption ΔL.

**Tabelle 2: Veränderung der Lichtabsorption ΔL**

| Proben lösung | ΔL [%] |
|---|---|
| E-I | 11 |
| E-II | 59 |
| E-III | 42 |
| E-IV | 31 |

### 2. In-vivo Test zur Antitranspirantwirkung

Zur Ermittlung der Antitranspirantwirkung wurde eine Antitranspirantstudie auf dem Rücken von 16 Probandinnen durchgeführt. Hierzu wurden die folgenden schweißhemmenden Mittel verwendet:

| Schweißhemmendes Mittel | Nr |
|---|---|
| Wässrige Lösung mit 10 % ACH, pH 4 | V-I |
| Wässrige Lösung mit 5 %* Protein ^{a)}, pH 2-4 | E-V** |
| Wässrige Lösung mit 5 %* Protein ^{b)}, pH 2-4 | E-VI** |
| Wässrige Lösung mit 5 %* Protein ^{c)}, pH 2-4 | E-VII** |
| Wässrige Lösung mit 5 %* Protein ^{d)}, pH 2-4 | E-VIII** |

| | |
|---|---|
| *Aktivsubstanz **erfindungsgemäß ^{a)} Hydrolactin QM LQ (WD); (INCI: Cocodimonium Hydroxypropyl Hydrolyzed Milk Protein; 20 Gew.-%ige Lösung in Wasser; Mw etwa 1.300 Da; Croda) ^{b)} Promois Milk; (INCI: Hydrolyzed Casein; 30 Gew.-%ige Lösung in Wasser; Mw etwa 600 Da; Seiwa Kasai CO. LTD) ^{c)} Hydrolactin 2500 PW (WD); (INCI: Hydrolyzed Milk Protein; 90% Gew.-%ige Lösung in Wasser; Mw etwa 1.800 Da; Croda) ^{d)} Yoghurt Protein GBU (INCI: Yoghurt; 35 Gew.-%ige Lösung in Wasser; M_{w} etwa 3.000 bis 35.000 Da; Cosmetochem) | |

Auf dem Rücken von 16 Probandinnen wurden jeweils auf einer Seite neben dem Rückgrat 40 µL des schweißhemmenden Mittels V-I und 75 µL der kosmetischen Mittel E-V, E-VI, E-VII und E-VIII aufgetragen. Nach 5 Minuten wurden die behandelten Stellen mit okklusiver nichtadsorbierender Folie abgedeckt. Nach 2 Stunden wurden diese nichtadorbierenden Pads entfernt. Die Zusammensetzungen wurden an vier aufeinanderfolgenden Tagen jeweils auf die zuvor beschriebene Art und Weise auf den Rücken der Probandinnen aufgetragen. 24 h nach dem letzten Auftragen der Zusammensetzung wurden auf dem Rücken der Probandinnen saugfähige Pads an den Stellen aufgebracht, wo zuvor die Zusammensetzungen appliziert wurden. Weiterhin wurden auf der anderen Seite des Rückgrats in der gleichen Höhe ebenfalls Pads aufgebracht, welche als Kontrolle dienten. Nachdem die Probandinnen für ca. 15 Minuten bei 80 °C in der Sauna geschwitzt hatten, wurde die Menge des durch die Pads aufgenommenen Schweißes gravimetrisch bestimmt, wobei jede Zusammensetzung mit der jeweils korrespondierenden unbehandelten Stelle auf dem Rücken verglichen wurde. Aus der gravimetrischen Bestimmung der Schweißmenge wurde die Schweißreduktion ermittelt, wobei alle ermittelten Werte statistisch signifikant waren.

Die Schweißreduktion der jeweiligen Zusammensetzung im Vergleich zu einer unbehandelten Hautstelle ist in der nachfolgenden Tabelle wiedergegeben:

| Nr | Schweißreduktion |
|---|---|
| V-I | 50 % |
| E-V | 3 % |
| E-VI | 3 % |
| E-VII | 7 % |
| E-VIII | 22 % |

Die Verwendung des speziellen Proteins führt zu einer signifikanten Verminderung der Schweißreduktion und zu einer zufriedenstellenden Antitranspirantwirkung.

### 3. Formulierungen:

Das in den nachfolgenden Beispielen eingesetzte Milchprotein und/oder Joghurtprotein und/oder Seidenprotein bevorzugt ausgewählt aus folgenden Proteinen:
1) Hydrolysiertes Milchprotein mit mindestens einer Lauryldimoniumhydroxypropylgruppe und einem mittleren Molekulargewicht Mw von etwa 1.300 Da und
2) Hydrolysiertes Casein mit einem mittleren Molekulargewicht M_{w} von etwa 600 Da und
3) Hydrolysiertes Milchprotein mit einem mittleren Molekulargewicht Mw von etwa 1.800 Da und
4) Sprühgetrocknetes Joghurtprotein aus fettarmem Joghurt mit einem mittleren Molekulargewicht M_{w} von 3.000 bis 35.000 Da und
5) Hydrolysiertes Seidenprotein.

Schweißhemmende kosmetische Mittel mit einem pH von 2,5 bis 10,0 (Mengenangaben in Gew.-%)

| | 1 | 2 | 3 | 4 | 5 | 6 | 7 |
|---|---|---|---|---|---|---|---|
| Isopropylmyristat | 0,50 | 0,10 | 0,50 | 1,0 | 2,0 | 3,0 | 5,0 |
| Protein | 0,50 | 2,0 | 3,0 | 5,0 | 7,0 | 10 | 20 |
| Eumulgin B3 ^{a)} | 3,0 | 3,0 | 3,0 | 4,0 | 4,0 | 4,0 | 5,0 |
| Parfum | 0,10 | 0,20 | 0,30 | 0,30 | 0,50 | 0,8 | 1,0 |
| Konservierungsmittel | 0,50 | 0,50 | 0,50 | 0,80 | 0,80 | 1,5 | 2,0 |
| pH-Stellmittel | ad pH | ad pH | ad pH | ad pH | ad pH | ad pH | ad pH |
| Wasser | ad 100 | ad 100 | ad 100 | ad 100 | ad 100 | ad 100 | ad 100 |

| | | | | | | | |
|---|---|---|---|---|---|---|---|
| ^{a)} Eumulgin B3 (INCI: Ceteareth-30; BASF) | | | | | | | |

## Patentansprüche

1. Verwendung mindestens eines Proteins zur zumindest teilweisen Beeinflussung der Schweißdrüse(n), wobei das mindestens eine Protein in tierischen Sekreten, Sekreten von Insekten oder humanen Sekreten vorkommt und wobei das mindestens eine Protein bei einer pH-Wert Änderung von mindestens 0,5 in einem pH-Bereich von pH 4,0 bis pH 8,0, einer Temperatur von 20 °C bis 40 °C und einer Konzentration des Proteins von 0,001 bis 10 Gew.-%, bezogen auf das Gesamtgewicht der für die Bestimmung der Veränderung der Lichtabsorption verwendeten Probenmischung, eine Veränderung der Lichtabsorption (gemessen wie in der Beschreibung beschrieben) von 1 bis 100 %, bewirkt, **dadurch gekennzeichnet, dass** das mindestens eine Protein ein Milchprotein und/oder ein Joghurtprotein und/oder ein Seidenprotein ist, wobei das Protein gegebenenfalls hydrolysiert ist und/oder wobei das Protein gegebenenfalls mindestens eine Lauryldimoniumhydroxypropylgruppe enthält.

2. Verwendung nach Anspruch 1, **dadurch gekennzeichnet, dass** das mindestens eine Protein in einer Gesamtmenge von 0,5 bis 60 Gew.-%, vorzugsweise von 1,5 bis 50 Gew.-%, bevorzugt von 2 bis 40 Gew-%, weiter bevorzugt von 2,5 bis 30 Gew.-%, insbesondere von 3 bis 20 Gew.-%, bezogen auf das Gesamtgewicht des schweißhemmenden kosmetischen Mittels, verwendet wird.

3. Verwendung nach einem der Ansprüche 1 oder 2, **dadurch gekennzeichnet, dass** das mindestens eine Protein ein mittleres Molekulargewicht M_{w} von 150 bis 100.000 Da, vorzugsweise von 180 bis 50.000 Da, bevorzugt von 200 bis 10.000 Da, weiter bevorzugt von 250 bis 8.000 Da, insbesondere von 300 bis 5.000 Da, aufweist.

4. Verwendung nach einem der vorangehenden Ansprüche, **dadurch gekennzeichnet, dass** das mindestens eine Protein Lysin in 1,0 bis 8,0 Mol.-%, Histidin in 1,0 bis 2,5 Mol.-% und Arginin in 2,0 bis 3,0 Mol.-%, bezogen auf die Gesamtstoffmenge aller Aminosäuren des Proteins, enthält.

5. Verwendung nach einem der vorangehenden Ansprüche, **dadurch gekennzeichnet, dass** das mindestens eine Protein einen Calciumgehalt von mindestens 1000 bis 15.000 mg, einen Kaliumgehalt von 400 bis 18.000 mg, einen Phosphorgehalt von 6.000 bis 11.000 mg und einen Schwefelgehalt von 3.000 bis 6.000 mg, jeweils bezogen auf 1 kg des mindestens einen Proteins, aufweist.

## Claims

1. The use of at least one protein for at least partially influencing the sweat gland(s), the at least one protein occurring in animal secretions, secretions of insects, or human secretions, and the at least one protein bringing about a change in light absorption (measured as described in the description) from 1 to 100% at a pH change of at least 0.5 in a pH range of from pH 4.0 to pH 8.0, at a temperature of from 20 °C to 40 °C, and at a concentration of the protein of from 0.001 to 10 wt.%, based on the total weight of the sample mixture used for determining the change in light absorption, **characterized in that** the at least one protein is a milk protein and/or a yogurt protein and/or a silk protein, the protein optionally being hydrolyzed and/or the protein optionally containing at least one lauryldimonium hydroxypropyl group.

2. The use according to claim 1, **characterized in that** the at least one protein is used in a total amount of from 0.5 to 60 wt.%, preferably from 1.5 to 50 wt.%, more preferably from 2 to 40 wt.%, even more preferably from 2.5 to 30 wt.%, in particular from 3 to 20 wt.%, based on the total weight of the antiperspirant cosmetic agent.

3. The use according to one of claims 1 or 2, **characterized in that** the at least one protein has an average molecular weight Mw of from 150 to 100,000 Da, preferably from 180 to 50,000 Da, more preferably from 200 to 10,000 Da, even more preferably from 250 to 8,000 Da, in particular from 300 to 5,000 Da.

4. The use according to one of the preceding claims, **characterized in that** the at least one protein contains lysine in an amount of 1.0 to 8.0 mol.%, histidine in an amount of 1.0 to 2.5 mol.%, and arginine in an amount of 2.0 to 3.0 mol.%, based on the total amount of all the amino acids of the protein.

5. The use according to one of the preceding claims, **characterized in that** the at least one protein has a calcium content of at least 1,000 to 15,000 mg, a potassium content of 400 to 18,000 mg, a phosphorus content of 6,000 to 11,000 mg, and a sulfur content of 3,000 to 6,000 mg, in each case based on 1 kg of the at least one protein.

## Revendications

1. Utilisation d'au moins une protéine pour influencer au moins partiellement l'au moins une glande sudoripare, l'au moins une protéine étant présente dans des sécrétions animales, des sécrétions d'insectes ou des sécrétions humaines, et l'au moins une protéine entraînant une modification de l'absorption de lumière (mesurée selon la description) de 1 à 100 % à une variation de pH d'au moins 0,5 dans une plage de pH comprise entre 4,0 et 8,0, une température comprise entre 20 et 40 °C et une concentration de la protéine comprise entre 0,001 et 10 % en poids, par rapport au poids total du mélange d'échantillons utilisé pour déterminer la modification de l'absorption de lumière, **caractérisée en ce que** l'au moins une protéine est une protéine laitière et/ou une protéine de yaourt et/ou une protéine de soie, la protéine étant éventuellement hydrolysée et/ou la protéine contenant éventuellement au moins un groupe lauryl dimonium hydroxypropyl.

2. Utilisation selon la revendication 1, **caractérisée en ce que** l'au moins une protéine est utilisée en une quantité totale comprise entre 0,5 et 60 % en poids, de préférence entre 1,5 et 50 % en poids, préférablement entre 2 et 40 % en poids, de manière davantage préférée entre 2,5 et 30 % en poids, en particulier entre 3 et 20 % en poids, par rapport au poids total de la composition cosmétique antisudorale.

3. Utilisation selon l'une des revendications 1 ou 2, **caractérisée en ce que** l'au moins une protéine présente un poids moléculaire moyen M_{w} compris entre 150 et 100 000 Da, de préférence entre 180 et 50 000 Da, préférablement entre 200 et 10 000 Da, de manière davantage préférée entre 250 et 8 000 Da, en particulier entre 300 et 5 000 Da.

4. Utilisation selon l'une des revendications précédentes, **caractérisée en ce que** l'au moins une protéine contient 1,0 à 8,0 % en mol de lysine, 1,0 à 2,5 % en mol d'histidine et 2,0 à 3,0 % en mol d'arginine, par rapport à la quantité totale de matière de tous les acides aminés de la protéine.

5. Utilisation selon l'une des revendications précédentes, **caractérisée en ce que** l'au moins une protéine présente une teneur en calcium comprise entre 1 000 et 15 000 mg, une teneur en potassium comprise entre 400 et 18 000 mg, une teneur en phosphore comprise entre 6 000 et 11 000 mg et une teneur en soufre comprise 3 000 et 6 000 mg, respectivement par rapport à 1 kg de l'au moins une protéine.
